# EUROPÄISCHE PATENTANMELDUNG

(11) **EP 0 960 594 A1**
(43) Veröffentlichungstag der Anmeldung: **01.12.1999**
(21) Anmeldenummer: 98250175.1
(22) Anmeldetag: 25.05.1998
(51) Int. Cl.: A47L 9/18, B01D 47/02

(54) **Luftreiniger**

(71) Anmelder: Erdogan, Hasan, 63128 Dietzenbach (DE)
(72) Erfinder: Erdogan, Hasan, 63128 Dietzenbach (DE)

(57) **Zusammenfassung**

Die Erfindung betrifft eine Luftreinigung durch Wasser - Filter - System.

Zu diesem Zweck sind vorgesehen, ein Behälter und ein Deckel.

Der Behälter wird bis zur Hälfte mit Wasser gefüllt.
Der Deckel ist mit einem Rohr und einem Entlüfter versehen.

Durch das Rohr wird die Außenluft ins Wasser geströmt, wo die Luft im Behälter mit dem Entlüfter ausgesaugt wird

## Beschreibung

Das Gerät ist mit einem Behälter und einem Deckel, der mit einem Rohr und einem Entlüfter ausgerüstet ist.

Der Behälter wird bis zur Hälfte mit Wasser aufgefüllt und mit dem Deckel verschlossen.

Das Rohr muß so lang sein, daß das Ende des Rohres ins Wasser taucht. Der Entlüfter leitet den Luft im Behälter nach außen.

Durch das absaugen der im Behälter vorhandene Innenluft strömt die Außenluft durch das Rohr ins Wasser und durch Wasser gefiltert ins Behälter, durch absaugen der Luft mit dem Entlüfter nach außen, so können Staub und Mikroorganismen die im Luft vorhanden sind, durch das Wasser nicht raus und bleiben durch diese Zirkulation im Wasser und somit wird die Reinigung der Luft erreicht.

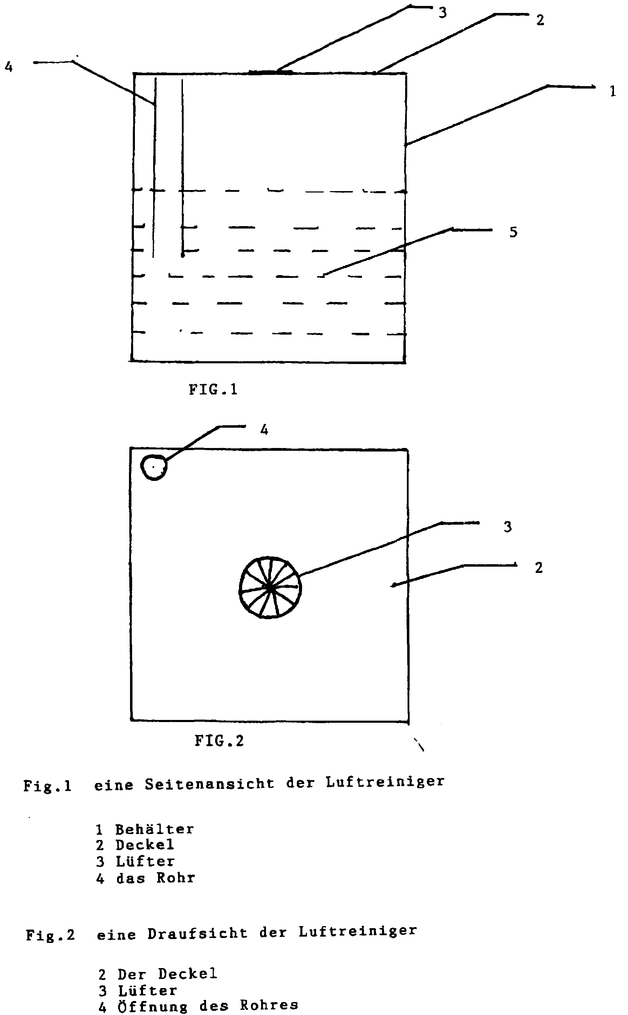

## Patentansprüche

1. Luftreinigung mit Wasser - Filter - System, dadurch gekennzeichnet, daß
Ein Behälter (1) bis zur Hälfte mit Wasser (5) gefüllt wird, dies zur Filterung dient.
Der Deckel (2) mit dem Rohr (4) saugt den Außenluft.
Durch den Entlüfter (3) der ebenfalls am Deckel montiert ist, wird der im Wasser bereits gefilterte Innenluft nach außen geströmt.
Durch diese Zirkulation wird der Luft gereinigt.

## Geänderte Patentansprüche

### Geänderte Patentansprüche gemäss Regel 86(2) EPÜ.

1. Luftreinigung mit Wasser - Filter - System für privatem Haushalt.
Luftreinigungsgerät für privatem Haushalt besteht aus zwei Teilen, dadurch gekennzeichnet, daß obere Teil des Gerätes (1) mit Röhren (2) ausgerüstet ist, die für die Lufteinströmung dienen und in den halbgefüllten Wasserbecken(9) (untere Teil des Gerätes) hineinragen. Der Motor (3) mit Gebläse(4) und Schaufelrad (5) ist auf dem unteren Deckel (7) montiert. Durch die Öffnung von Gebläse und Deckel(6) wird das Luft in halbgefülltem Wasserbecken abgesaugt, somit wird Unterdruck hergestellt, durch den entstandene Unterdruck strömt die Außenluft durch die Lufteinströmungsöffnungen(11) mit den Röhren(2) ins Wasser und wird gefiltert. Durch die seitlichen Öffnungen (8) wird die gereinigte Luft nach außen geströmt.
Um das Gerät parallel zur Erdoberfläche zu halten wird ein Wasserwaage (12) an das oberen Teil angebaut und an das untere Teil werden einstellbare Schraubenfüße (10) montiert.
